# EUROPEAN PATENT APPLICATION

(11) **EP 3 738 550 A1**
(43) Date of publication of application: **18.11.2020**
(21) Application number: 20184455.2
(22) Date of filing: 12.05.2010
(51) Int. Cl.: A61F 2/12, A61F 2/00, B29C 41/00

(54) **IMPLANTS AND METHODS FOR MANUFACTURING SAME**

(30) Priority: 13.05.2009 US 17795509 P
(62) Divisional of application: 10718827.8
(71) Applicant: Allergan, Inc., Irvine, CA 92612 (US)
(72) Inventor: Guillen, Blanca, Goleta, California 93117 (US); Stroumpoulis, Dimitrios, Goleta, California 93117 (US)
(74) Representative: Hoffmann Eitle

(57) **Abstract**

A implantable prosthesis having an external surface at least a portion of which is an open-cell structure is made by providing an implantable member having a surface applying a first layer (32) of elastomer to the surface, applying a first layer of particles (30) to the first layer of elastomer, applying a second layer (34) of elastomer to the first layer of particles (36), applying a second layer of particles to the second layer of elastomer, curing the layers of elastomer, and removing the particles to form an external surface at least a portion of which is an open-cell structure.

## Description

### RELATED APPLICATION

This application claims the benefit of U.S. Provisional Patent Application No. 61/177,955, filed on May 13, 2009, the entire disclosure of which is incorporated herein by this specific reference.

### BACKGROUND

The present invention generally relates to prosthetic implants and more specifically relates to implants having open-cell surfaces and methods for manufacturing same.

Capsular contracture is a relatively common complication in patients with breast implants. Encapsulation is a defensive mechanism of the body to the presence of a foreign object. Similar to the formation of scar tissue in the healing of a wound, encapsulation involves the formation of a fibrous tissue capsule around and enclosing a foreign object in the body, for example, an implanted prosthesis.

In some instances, and for reasons not well understood, the fibrous capsule begins to harden and contract, squeezing the implant into a nearly spherical shape. In such cases, the implanted breast can become painful and misshapen. Capsular contracture can be a serious condition both from medical and aesthetic viewpoints.

One way to remedy capsular contracture is to surgically remove the contracted capsule and implant and then insert either the same or another implant, a procedure called surgical capsulotomy or capsulectomy. Alternatively, some doctors use closed capsulotomy, a method wherein force is applied to break the capsule in situ.

The problem of capsular contracture is very complex and the reasons why it occurs are not yet fully understood. Nonetheless, several different approaches to avoiding the occurrence of capsular contraction have been investigated.

One approach in reducing the possibility of capsular contracture focuses on the collagen capsule itself, and on methods that can disrupt its organization, thickness and strength. Implants which include a textured surface have been found to be somewhat successful in this respect. Although the cause for reduced incidence of capsular contraction with textured surfaced implants is not fully understood, it is believed that the growth of fibrous tissue onto textured surfaces in many different directions reduces the density of the fibrous tissue and prevents it from contracting in a concerted manner.

There still remains a need for implants that are more effective in eliminating or at least reducing the incidence of capsular contraction.

### SUMMARY OF THE INVENTION

The present invention provides methods for manufacturing implantable prostheses having an external surface at least a portion of which is an open-cell structure, and prostheses made by such methods.

The prostheses formed by the present methods may reduce the incidence of capsular contraction.

In one aspect of the invention, the method comprises the steps of (a) providing an implantable member having a surface, (b) applying a first layer of elastomer to at least a portion of the surface of the member, and (c) applying a first layer of particles to the first layer of elastomer. The method further comprises the steps of (d) applying at least one additional layer of elastomer to the first layer of particles, (e) applying a second layer of particles to the second layer of elastomer, (f) curing the layers of elastomer, and (g) removing the particles from the member to form an external surface, at least a portion of which is an open-cell structure.

The step of removing the particles may comprise causing the particles to dissolve or contacting the particles with an abrasive surface.

In one aspect of the invention, the particles comprise solid particles, for example, solid crystal particles, for example, salt crystals. The crystals may be substantially cubic or rounded, or may comprise a mixture of substantially cubic and rounded. The particles have an average particle size distribution in the range of about 100 microns to about 800 microns. In some embodiments, salt powder is mixed with relatively larger salt crystals, the powder having an average particle size of less than 100 microns, for example, about 10 microns.

In some embodiments of the invention, each of the first and second layers of particles is made up of substantially uniformly sized/shaped particles.

In another aspect of the invention, each of the first and second layers of particles is made up of differently sized or shaped components.

For example, the first layer of particles may be comprised of a first component of relatively large particles as well as a second component of relatively smaller particles distributed therein. When removed from the first elastomer layer, the particles leave highly interconnected pores.

In some embodiments, the particles comprise a mixture of solid particles including a first component and a second component different from the first component. For example, the first component may be made up of particles having a mean particle size distribution in the range of about 400 to about 800 microns, and the second component may be made up of particles having a mean particle size distribution in the range of about 100 to about 300 microns, or less than 100 microns.

In other embodiments, the first and second components are made up of salt crystals and salt powder, respectively.

In yet other embodiments, the first and second components are made up of substantially cubic crystals and rounded crystals, respectively.

In still other embodiments, the first and second components are made up of salt crystals and stabilized water droplets, respectively.

In one aspect of the invention, the method includes the step of increasing contact between the particles, for example, within each layer and/or between each layer. For example, in some embodiments, the method includes the step of causing the particles within each layer to fuse together. In some embodiments, the method includes the step of adding an emulsion to the layer of elastomer to cause particles in one layer to contact particles in an adjacent layer.

In another aspect of the invention, a method of making an implantable prosthesis is provided which generally comprises the steps of forming a layered salt scaffold comprising fused salt particles and then applying an elastomer dispersion thereto. For example, a method for making an implantable prosthesis in accordance with the invention may comprise the steps of:
(a) providing a surface;
(b) applying a first layer of particles to the surface;
(c) causing the particles of the first layer of particles to at least partially fuse together;
(d) applying a second layer of particles to the at least partially fused first layer of particles;
(e) causing the particles of the second layer of particles to at least partially fuse together and with the particles of the first layer;
(f) applying an elastomer to the at least partially fused first and second layers of particles;
(g) curing the elastomer; and
(h) removing the particles from the cured elastomer.

The method may also include the step of applying a vacuum to the layers of particles and elastomer to enhance contact between the elastomer and the particles, before the step of curing the elastomer.

The method may comprises repeating steps (d) and (e) with a third, fourth, fifth, or additional layer of particles prior to the step of applying an elastomer.

In a specific embodiment, the scaffold is formed of four layers of fused salt particles. The particles are sized and shaped such that the four layers form a scaffold having a thickness of between about 0.5 mm and 3 mm. In one embodiment, the four layers form a scaffold having a thickness of about 1.5 mm. In this embodiment, the particles may be round particles and, more specifically may be rounded salt particles having a particle size of about 400 µm.

### BRIEF DESCRIPTION OF THE DRAWINGS

Aspects and advantages of the present invention will become better understood and appreciated with respect to the following detailed description and the accompanying drawings of which:
Fig. 1 is a side view of a mandrel useful in the methods of the present invention for forming a mammary prosthesis;
Fig. 2 is a rear view of the mandrel shown in Fig. 1;
Fig. 3 is a diagram illustrating a method for forming an elastomer surface of a prosthesis of the invention;
Fig. 4 shows four elastomers which have been prepared in accordance with various methods of the invention;
Figs. 5A and 5B are SEM micrographs of elastomers prepared in accordance with a method of the invention;
Fig. 6 is a diagram illustrating another method of the invention; and
Fig. 7 is a diagram illustrating yet another method in accordance with the invention.

### DETAILED DESCRIPTION

The present invention will primarily be described in the context of a mammary prosthesis, but is not limited thereto. The present invention is expected to help solve the problem of capsular contraction that is particularly troublesome in the implantation of mammary prostheses. It should be appreciated that the teachings of the present invention should prove to be advantageous wherever capsular contraction can damage a medical implant or cause discomfort to the patient and/or wherever a medical implant is to be anchored through the ingrowth of fibrous tissue. The present invention should also prove advantageous in preventing or controlling scar formation during wound healing after many types of plastic surgery.

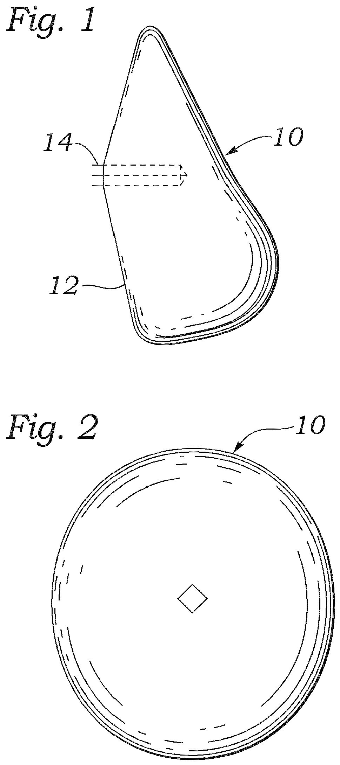

With reference to Figs. 1 and 2, a mandrel 10 has an external configuration corresponding to that of the mammary prosthesis to be formed by it. A rear face 12 of the mandrel has a support member 14 embedded therein and extending outward therefrom. As shown in Fig. 2, the support member enters the mandrel at the center of rear face 12. Mandrels of this type are standard in the field. They are typically made of Delrin, aluminum, stainless steel or plastics, such as Teflon/nylon combinations or high density polyethylene or polyester resin. A primary consideration is that the material be inert to the solvents and process heat used in the manufacturing process.

To begin the manufacture of a mammary prosthesis, the mandrel is dipped into a silicone rubber dispersion. Many such dispersions are used in the field. Typically, such dispersions contain a silicone elastomer and a solvent. The silicone elastomer may be polydimethylsiloxane, polydiphenyl-siloxane or some combination of these two. Typical solvents include xylene or trichloromethane. Different manufacturers vary the type and amount of the ingredients in the dispersion, the viscosity of the dispersion and the solid content of the dispersion. Nonetheless, the present invention is expected to be adaptable to have utility with a wide variety of silicone rubber dispersions.

The mandrel 10 is lowered into the dispersion while being supported by support member 14 until the mandrel is completely submerged. The mandrel 10 is then raised out of the dispersion with a thin coating of the material adhering thereto. The solvent in this thin coating is volatilized or caused to evaporate. Normally this is accomplished by flowing air over the coated mandrel at a controlled temperature and humidity. Different manufacturers use various quantities, velocities or directions of air flow and set the temperature and humidity of the air at different values. However, the desired result, driving off the solvent, remains the same. It is also common for prostheses manufacturers to repeat this dip and volatilize procedure a number of times so that a number of layers are built up on the mandrel to reach a desired shell thickness.

After the mandrel is raised out of the dispersion with an elastomer adhering thereto, the elastomer is allowed to stabilize. For example, the mandrel is held in a stable position until the final coating no longer flows freely. This occurs as some of the solvent evaporates from the final coating, raising its viscosity. As will be explained herein after, at this point in the method, layers of solid, soluble particles alternating with layers of elastomer are applied until a desired thickness is achieved.

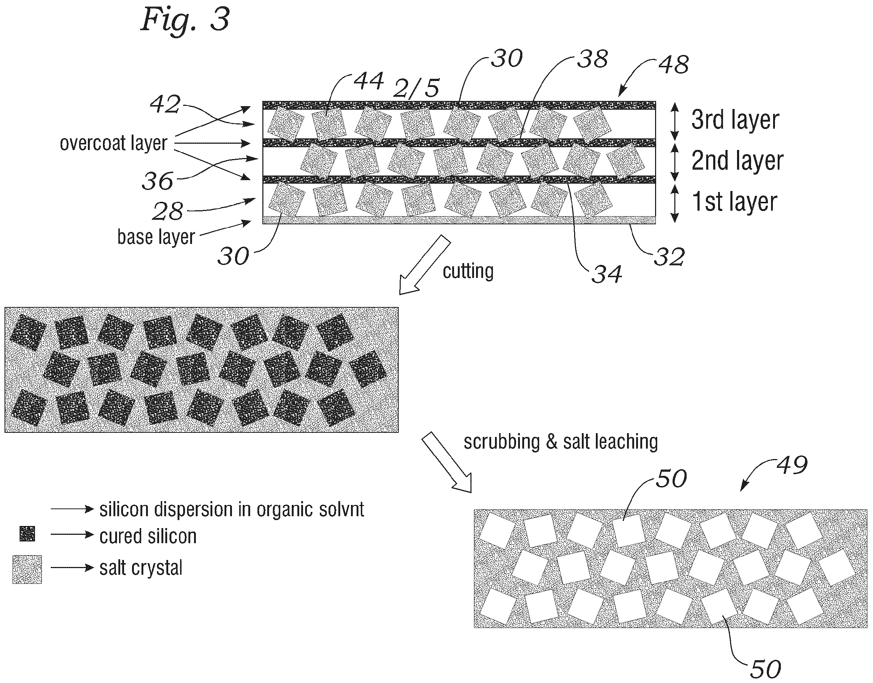

More specifically, turning now to Fig. 3, a schematic/flow diagram of a method in accordance with the present invention is illustrated. A layer 28 of particles 30, for example, granulated solid particles, for example, salt crystals, is applied to the stabilized elastomer layer 32.

The particles 30 may be applied to the elastomer layer 32 in any suitable manner, for example, by manually or mechanically sprinkling the particles 30 over the elastomer layer 32 while the mandrel (not shown in Fig. 3) is manipulated. Alternatively, a machine, for example, one which operates like a bead blaster or sand blaster, could be used to deliver a steady stream of solid particles at an adequate velocity to the elastomer on the mandrel. Other alternative methods for applying the particles are also possible within the scope of the present invention. For example, it is envisioned that adequate methods of particle application can be developed based on mechanisms that pour the particles onto the elastomer layer, or dip the elastomer coated mandrel into a body of the particles or contact the elastomer coated mandrel with a suspension of the particles.

Still referring to Fig. 3, another elastomer layer 34, for example, in a dispersion form, is applied to the first layer 28 of particles 30. This second layer 34 of elastomer may then be allowed to stabilize. Another layer 36 of particles 30 is applied to this second layer 34 of elastomer.

Optionally, a third layer 38 of elastomer is applied to the second layer 36 of particles 30 and a third layer 42 of particles 30 is applied to the third layer 38 of elastomer. Another layer 44, for example, a final layer, of elastomer may be applied to the third layer 42 of particles 30.

After application of the alternating particle layers 28, 36, 42 and elastomer layers 32, 34, 38, 44, the elastomer is then cured by subjecting the coated mandrel to suitable curing conditions. For example, depending upon the type of elastomer used, curing may be effected by placing the coated mandrel in an oven at elevated temperatures for example, between about 200°F and about 350°F for a suitable time, for example, between about 20 minutes and about 120 minutes.

After curing the elastomer, the layered elastomer/particle shell 48 can then be removed or stripped from the mandrel.

The particles are removed from the shell, either after or before the shell is removed from the mandrel. Removal of the particles leaves a textured shell 49, the external surface of which includes voids 50 or pores roughly made up of impressions left by the removed particles.

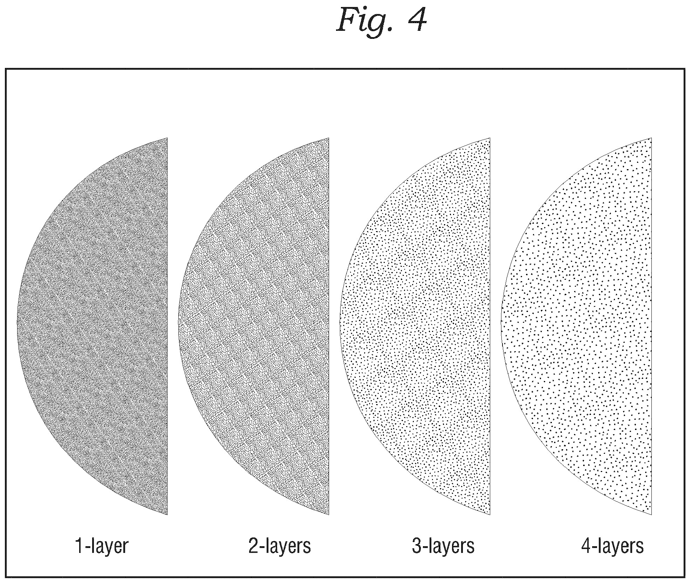

Fig. 4 shows photographs of textured elastomer shell surfaces made in accordance with the method described above using 1, 2, 3 and 4 alternating elastomer/particle layers, respectively.

Removal of the particles may be achieved by causing the particles to dissolve, for example, exposing the elastomer to a solvent for the particles. In some embodiments, the shell is placed in a solvent for the particles and agitated to ensure dissolution of all the particles. When the shell is removed from the solvent, the solvent is evaporated. Alternatively or additionally, the particles may be removed by contacting the particles with an abrasive surface and sloughing the particles from the elastomeric layers.

The particles may comprise a salt which is readily available in granulated form. In some embodiments, the particles are a material selected from the group consisting of sodium chloride, barium sulfate, potassium nitrate, sodium carbonate, and mixtures thereof.

In a particular embodiment, the particles are sodium chloride. The solvent may be water, which readily dissolves sodium chloride and does not dissolve silicone rubber. However, the person skilled in the art will understand that a number of solid and solvent pairs could be chosen that would more or less fulfill the above stated requirements.

In one aspect of the invention, the method further comprises the step of causing at least some of the particles to fuse together prior to the steps of curing the elastomers and removing the particles. In this embodiment, physical contact between the particles defines the interconnectivity of the pores. Interconnectivity can be enhanced by placing the coated mandrel in a humid environment or humidity chamber to cause the salt crystals to begin to dissolve on surfaces thereof, and fuse together.

In yet another embodiment, at least some of the particles in each particle layer are in contact with particles in adjacent particle layers (contact in a vertical plane). This too enhances interconnectivity of the pores formed upon removal of the particles.

Decreasing the viscosity of the elastomer layers will generally result in a thinner elastomer layer, which may be effective to improve physical contact between the particles between adjacent layers.

In another aspect of the invention, methods for manufacturing an implantable prosthesis are provided which generally comprise the steps providing an implantable member having a surface, applying a layer of elastomer to at least a portion of the surface of the member, applying a first layer of particles to the first layer of elastomer, and causing at least some of the particles to fuse to one another. After the elastomer is cured, the particles are removed therefrom, thereby leaving highly interconnected pores. The particles may be caused to fuse to one another by exposing the particles to a suitable environment. In a specific embodiment, the particles embedded in the elastomer are sodium chloride crystals and they are exposed to a high humidity and elevated temperature, for example, 75% humidity at 37° C for about 24 hours to cause the desired fusion.

After finishing the shell according to the steps described above, the steps required to make a finished mammary prosthesis may be conventional. First, the opening left by support member 14 is patched with uncured silicone elastomer sheeting. If the prosthesis is to be filled with silicone gel, this gel is added and cured, the filled prosthesis packaged, and the packaged prosthesis sterilized. If the prosthesis is to be inflated with a saline solution, a valve is assembled and installed, the prosthesis is post cured if required, and the prosthesis is then cleaned, packaged and sterilized. A combination silicone/saline mammary prosthesis can also be made.

A method has been described for creating an outer layer having an open-cell structure in a silicone elastomer member. More specifically, the method can be applied to create a medical implant with an external surface layer of silicone elastomer having an open-cell structure, to create strips having a textured surface for control of scar formation, or to improve a process for making mammary prostheses. The product made by this method has also been described and is expected to have great utility in preventing capsular contraction, in preventing or controlling scar formation, and in anchoring medical implants.

Scar tissue formation in the healing of a wound or surgical incision is also a process involving the growth of fibrous tissue. A visible scar results from this healing process because the fibrous tissue is aligned in one direction. However, it is often aesthetically desirable to prevent scar formation, especially in certain types of plastic surgery. A member having an open-cell structure surface made in accordance with the present invention can be placed subcutaneously within a healing wound or incision to prevent the fibrous tissue from aligning and thereby prevent or reduce scar formation.

It is often important to anchor medical implants against movement. Mammary prostheses are one example of implants that must be anchored. Facial implants are another example of implants that must be anchored. With facial implants it is particularly important that they be anchored securely against movement because of their prominent location. Providing such implants with an open-cell structure surface made in accordance with the present invention is a particularly advantageous way to ensure that they will be anchored securely.

### EXAMPLE 1

### Preparation of silicone foam coated elastomers using layering and a single salt crystal type technique

Silicone foam coated elastomers are prepared using a salt multi-layer technique in accordance with the invention. In this approach each pore is formed by salt crystals and therefore the pore shape and size is generally defined by the shape and size of the salt crystal used. One type of crystals appropriate for this application is cubic, such that the edges of each crystal can come in physical contact with neighboring crystals forming interconnections. Other types can be also used, e.g. round, elongated or even fused crystals of more complex geometry.

A thin base layer (tack layer) is first applied on top of the elastomer of interest, to provide adhesion. That layer is a low viscosity silicone dispersion (e.g. polydimethylsiloxane, polydiphenylsiloxane, etc.) in an organic solvent (e.g. xylene). The layer is allowed to evaporate most of the solvent off.

Salt crystals are added on top of the base layer forming a closely packed single layer. The latter is achieved by firmly compressing the crystals against excess salt.

The crystals are partially fused by incubating the sample in an environmental chamber at about 37°C and about 75% humidity for about 24 hrs.

The sample is allowed to dry and a thin overcoat layer is applied on top of the salt layer. This is typically a very low viscosity silicone dispersion. The layer is allowed to evaporate most of the solvent off.

The process is repeated as many times as desired to produce 2, 3, 4, ...n layers of salt and elastomer.

The elastomer is allowed to evaporate the solvent off and subsequently cured at approximately 145°C.

The top layer is scrubbed against a rough surface to break open the outermost pores and submerged into warm water to dissolve and wash-off the salt.

Alternatively the process can be completed with a salt layer on the top. This may facilitate the salt leaching process and avoid the use of mechanical abrasion (scrubbing).

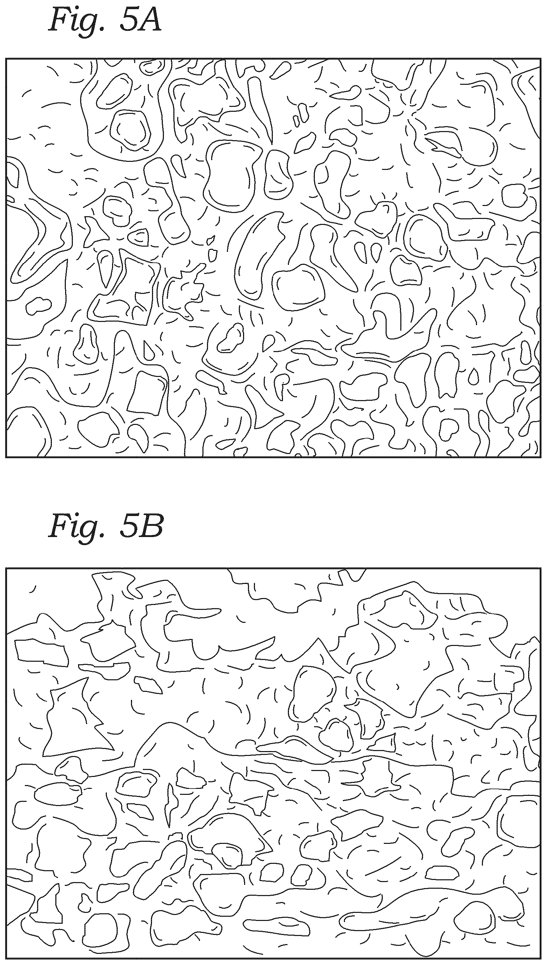

Fig. 5A and 5B are SEM micrographs (top view and cross sectional view respectively) of elastomers prepared according to Example 1.

### EXAMPLE 2

### Preparation of silicone foam coated elastomers using a dual salt crystal type technique

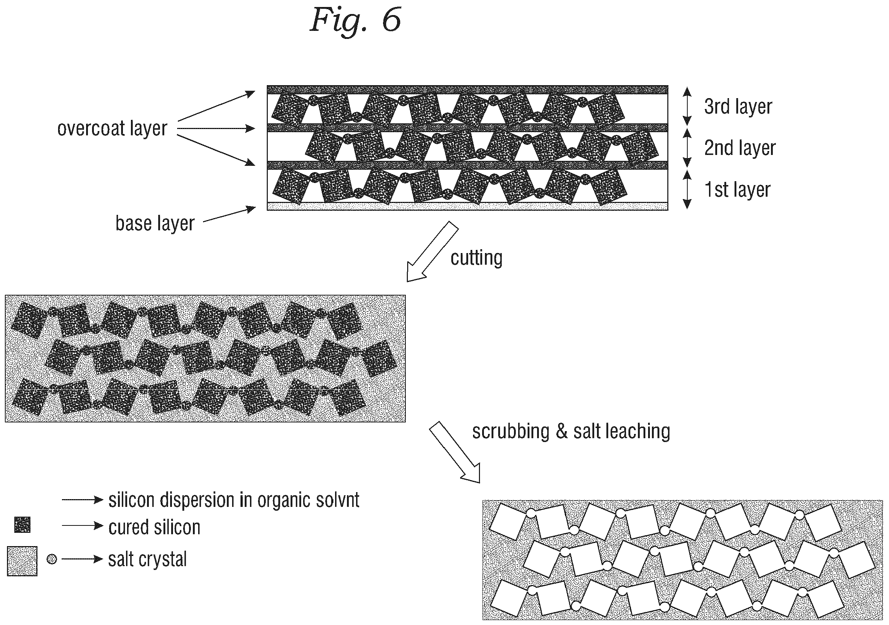

Fig. 6 illustrates another method in accordance with the present invention. This method may be identical to the method described in Example 1, with the exception that smaller crystals are also used to enhance the contact area between the large crystals, thus forming more and bigger interconnections. The smaller crystals can have a round, square or more complex geometry. The ratio of large to small crystals can vary, based on the geometry. If the large crystals are round, a theoretical maximum packing density will be about π/√18 ≈ 0.74, and the mixing ratio about 3:1 or higher (large:small crystals). If the large crystals are cubic, the theoretical maximum packing density will be 1 and typically a minimum amount of small crystals will be required.

### EXAMPLE 3

### Preparation of silicone foam coated elastomers using a single salt crystal type emulsion technique

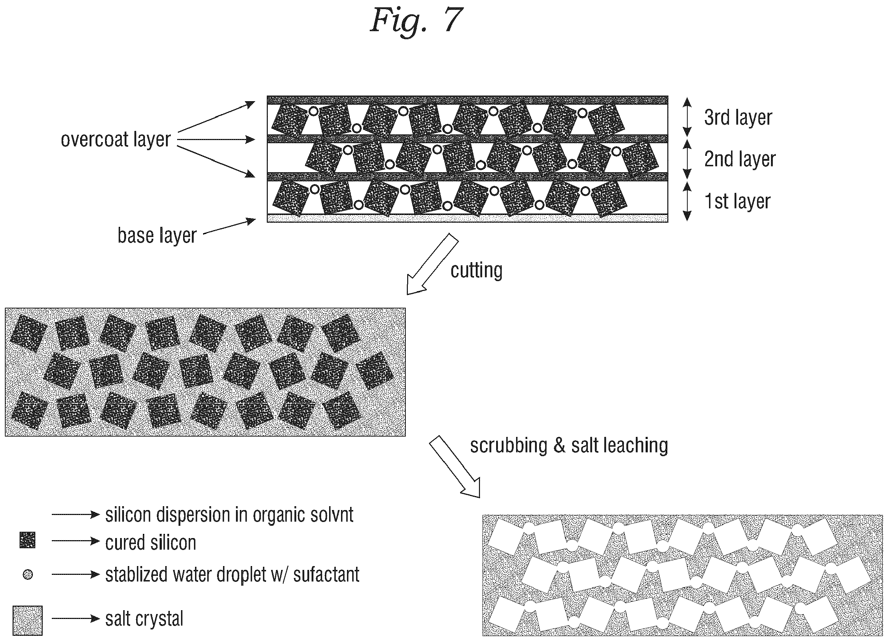

Fig. 7 illustrates yet another method in accordance with the present invention. In this approach, the technique described in Example 2 is modified to replace the smaller crystals with stabilized water droplets. The advantage of this substitution is that the water droplets can selectively wet neighboring large crystals, dissolving part of the salt and reshaping it into a bridge that connects the neighboring crystals. Emulsion techniques can be used to stabilize water droplets in organic solvents, using surfactants (monoalkyl or dialkyl ampliphilic molecules). The stabilized water droplets can be added to the silicone dispersion, the base layer, the overcoat layer or any combination of those three.

While this invention has been described with respect to various specific examples and embodiments, it is to be understood that the invention is not limited thereto and that it can be variously practiced within the scope of the invention.

### Preferred embodiments of the invention:

1. A implantable prosthesis having an external surface at least a portion of which is an open-cell structure, the prosthesis made by the method comprising the steps of:
   (a) providing an implantable member having a surface;
   (b) applying a first layer of elastomer to at least a portion of the surface of the member;
   (c) applying a first layer of particles to the first layer of elastomer;
   (d) applying a second layer of elastomer to the first layer of particles;
   (e) applying a second layer of particles to the second layer of elastomer;
   (f) curing the layers of elastomer; and
   (g) removing the particles to form an external surface at least a portion of which is an open-cell structure.
2. The prosthesis of embodiment 1 further comprising the step of applying a third layer of elastomer to the second layer of particles before the step of removing the particles.
3. The prosthesis of embodiment 1 wherein the step of removing the particles comprises causing the particles to dissolve.
4. The prosthesis of embodiment 1 wherein the step of removing comprises contacting the particles with an abrasive surface.
5. The prosthesis of embodiment 1 further comprising causing at least some of the particles to fuse together prior to the step of removing the particles.
6. The prosthesis of embodiment 1 further comprising causing at least some of the particles in the first particle layer to contact some of the particles in the second particle layer.
7. The prosthesis of embodiment 1 wherein the particles comprise solid particles.
8. The prosthesis of embodiment 1 wherein the particles comprise angular particles.
9. The prosthesis of embodiment 1 wherein the particles comprise substantially cubic particles.
10. The prosthesis of embodiment 1 wherein the particles comprise substantially rounded particles.
11. The prosthesis of embodiment 1 wherein the particles have a particle size distribution in the range of about 100 microns to about 800 microns.
12. The prosthesis of embodiment 1 wherein the particles are comprised of a first component comprising particles having a first size range and a second component comprising particles having a second size range that is different than the first size range.
13. The prosthesis of embodiment 1 wherein the particles are comprised of a first component comprising salt particles and a second component comprising water droplets.
14. The prosthesis of embodiment 1 wherein the particles comprise a first component having a particle size distribution in the range of about 400 to about 800 microns and a second component having a particle size distribution in the range of about 100 to about 300 microns.
15. The prosthesis of embodiment 14 wherein the first component comprises salt crystals having a particle size distribution of between about 100 to about 800 microns, and the second component comprises salt powder having a particle size distribution of less than 100 microns.
16. The prosthesis of embodiment 15 wherein the second component comprises salt powder having a particle size distribution of about 10 microns.
17. The prosthesis of embodiment 10 wherein the first component comprises substantially cubic particles and the second component comprises substantially rounded particles.
18. A implantable prosthesis having an external surface at least a portion of which is an open-cell structure, the prosthesis made by the method comprising the steps of:
   (a) providing an implantable member having a surface;
   (b) applying a layer of elastomer to at least a portion of the surface of the member;
   (c) applying a layer of particles to the layer of elastomer;
   (d) causing at least some of the salt particles to fuse to one another;
   (e) curing the elastomer; and
   (f) removing the particles to form an external surface at least a portion of which is an open-cell structure.
19. The prosthesis of embodiment 18 wherein the step of causing at least some of the particles to fuse to one another comprises exposing the particles to an environment having a humidity sufficient to cause the particles to fuse to one another.
20. The prosthesis of embodiment 19 wherein the humidity is about 75%.
21. The prosthesis of embodiment 20 wherein the environment is at a temperature of about 37°C.
22. A method of making an implantable prosthesis having an external surface at least a portion of which is an open-cell structure, the method comprising the steps of:
   (a) providing an implantable member having a surface;
   (b) applying a first layer of elastomer to at least a portion of the surface of the member;
   (c) applying a first layer of particles to the first layer of elastomer;
   (d) applying a second layer of elastomer to the first layer of particles;
   (e) applying a second layer of particles to the second layer of elastomer;
   (f) curing the layers of elastomer; and
   (g) removing the particles to form an external surface at least a portion of which is an open-cell structure.
23. A method of making an implantable prosthesis comprising:
   (a) providing a surface;
   (b) applying a first layer of particles to the surface;
   (c) causing the particles of the first layer of particles to at least partially fuse together;
   (d) applying a second layer of particles to the at least partially fused first layer of particles;
   (e) causing the particles of the second layer of particles to at least partially fuse together;
   (f) applying an elastomer to the at least partially fused first and second layers of particles;
   (g) curing the elastomer; and
   (h) removing the particles.
24. The method of embodiment 23, further comprising the step of:
   applying a vacuum to the layers of particles and elastomer before the step of curing the elastomer.
25. The method of embodiment 23 further comprising repeating steps (d) and (e) with a third layer of particles prior to the step of applying an elastomer.
26. The method of embodiment 25 further comprising repeating steps (d) and (e) with a fourth layer of particles prior to the step of applying an elastomer.
27. The method of embodiment 23 wherein the particles are sized and shaped such that the four layers form a scaffold having a thickness of about 1.5 mm.
28. The method of embodiment 23 wherein steps (d) and (e) are repeated with additional layers of particles until the layers of particles form a scaffold having a thickness of between about 0.5 mm and 3 mm.
29. The method of embodiment 23 wherein steps (d) and (e) are repeated until the layers of particles form a scaffold having a thickness between about 1.0 mm and 2.0 mm.
29. The method of embodiment 23 wherein steps (d) and (e) are repeated until the layers of particles form a scaffold having a thickness of about 1.5 mm.
30. The method of embodiment 23 wherein the particles have a particle size of about 400 µm.
31. The method of embodiment 23 wherein the particles are round particles.
32. The method of embodiment 23 wherein the particles are round particles having a size of about 400 µm.

## Claims

1. A implantable prosthesis having an external surface at least a portion of which is an open-cell structure, the prosthesis made by the method comprising the steps of:
(a) providing an implantable member having a surface;
(b) applying a first layer of elastomer to at least a portion of the surface of the member;
(c) applying a first layer of particles to the first layer of elastomer;
(d) applying a second layer of elastomer to the first layer of particles;
(e) applying a second layer of particles to the second layer of elastomer;
(f) curing the layers of elastomer; and
(g) removing the particles to form an external surface at least a portion of which is an open-cell structure,
wherein the particles are a material selected from the group consisting of sodium chloride, barium sulfate, potassium nitrate, sodium carbonate, and mixtures thereof.

2. The prosthesis of claim 1 further comprising the step of applying a third layer of elastomer to the second layer of particles before the step of removing the particles.

3. The prosthesis of claim 1 wherein the step of removing the particles comprises causing the particles to dissolve.

4. The prosthesis of claim 1 wherein the step of removing comprises contacting the particles with an abrasive surface.

5. The prosthesis of claim 1 further comprising causing at least some of the particles to fuse together prior to the step of removing the particles.

6. The prosthesis of claim 1 further comprising causing at least some of the particles in the first particle layer to contact some of the particles in the second particle layer.

7. The prosthesis of claim 1 wherein the particles comprise solid particles.

8. The prosthesis of claim 1 wherein the particles comprise (i) angular particles; (ii) substantially cubic particles; or (iii) substantially rounded particles.

9. The prosthesis of claim 1 wherein the particles have a particle size distribution in the range of about 100 microns to about 800 microns.

10. The prosthesis of claim 1 wherein the particles are comprised of a first component comprising particles having a first size range and a second component comprising particles having a second size range that is different than the first size range.

11. The prosthesis of claim 1 wherein the particles are comprised of a first component comprising salt particles and a second component comprising water droplets.

12. The prosthesis of claim 1 wherein the particles comprise a first component having a particle size distribution in the range of about 400 to about 800 microns and a second component having a particle size distribution in the range of about 100 to about 300 microns.

13. The prosthesis of claim 12 wherein the first component comprises salt crystals having a particle size distribution of between about 100 to about 800 microns, and the second component comprises salt powder having a particle size distribution of less than 100 microns.

14. The prosthesis of claim 13 wherein the second component comprises salt powder having a particle size distribution of about 10 microns.

15. The prosthesis of claim 12 wherein the first component comprises substantially cubic particles and the second component comprises substantially rounded particles.
